# EUROPÄISCHE PATENTSCHRIFT

(11) **EP 2 216 645 B1**
(45) Veröffentlichungstag und Bekanntmachung des Hinweises auf die Patenterteilung: **10.10.2012**
(21) Anmeldenummer: 09177503.1
(22) Anmeldetag: 30.11.2009
(51) Int. Cl.: G01N 22/04, G01N 33/34

(54) **Verfahren und Vorrichtung zur Bestimmung des Feuchtegehalts von Altpapier**
Method and device for calculating the moisture content of recycled paper
Procédé et dispositif de détermination du taux d'humidité de vieux papier

(30) Priorität: 04.02.2009 DE 102009007362
(43) Veröffentlichungstag der Anmeldung: 11.08.2010
(73) Patentinhaber: Siemens Aktiengesellschaft, 80333 München (DE)
(72) Erfinder: Ehben, Thomas, 91085, Weisendorf (DE); Mielke, Jürgen, 96049, Bamberg (DE)

(56) Entgegenhaltungen:
- WO-A1-03/058236
- DE-A1- 1 549 232
- DE-A1- 3 317 200
- DE-A1- 3 905 658
- DE-T2- 69 729 117
- JP-A- 61 034 448

## Beschreibung

Die Erfindung betrifft ein Verfahren und eine Vorrichtung zur Bestimmung des Feuchtegehalts von Altpapier bzw. einer Altpapiercharge.

Angesichts der weltweit steigenden Papierproduktion entwickelt sich Altpapier zu einem zunehmend begehrten Rohstoff. Der das Altpapier abnehmende Papiererzeuger oder andere Abnehmer zahlen in der Regel an einen Altpapierlieferanten eine mengenabhängige Vergütung, die sich am Welt-Altpapiermarkt orientiert. Die Bestimmung der angemessenen Vergütung für das angelieferte Altpapier gewinnt dabei stetig an Bedeutung. Die Altpapiermenge wird in der Regel bei Anlieferung durch Wiegen bestimmt. Dabei bringt die gleiche Menge Altpapier in Abhängigkeit vom Feuchtegehalt des Altpapiers stark voneinander unterschiedliche Massen auf die Waage. Die vom Papiererzeuger bzw. Abnehmer zu zahlende Vergütung ist in diesem Fall somit vom Feuchtegehalt des Altpapiers abhängig.

Eine Trocknung des angelieferten Altpapiers ist technisch und betriebswirtschaftlich nicht sinnvoll.

Daher sind bereits Vorrichtungen und Verfahren zur Ermittlung des Feuchtegehalts in Altpapier vorgeschlagen worden, um eine vom Feuchtegehalt unabhängige Vergütung vornehmen zu können.

Ein solches Verfahren und eine solche Vorrichtung sind aus JP 61034448 A bekannt. Die hier beschriebene Vorrichtung weist eine Sendeantenne zum Aussenden von Mikrowellen und eine Empfangsantenne zu deren Empfang auf. Zwischen der Sende- und der Empfangsantenne wird komprimiertes Altpapier angeordnet und dieses mit Mikrowellenstrahlung beaufschlagt. Je nach Feuchtegehalt des komprimierten Altpapiers erfolgt eine mehr oder weniger starke Dämpfung der Mikrowellensignale. Die Dämpfung wird mit einem, an einer Standardprobe mit bekanntem Feuchtegehalt ermittelten Dämpfungswert verglichen. Aus der Differenz der beiden Dämpfungswerte lässt sich der Feuchtegehalt für das jeweils zu messende Altpapier bestimmen.

DE 697 29 117 T2 beschreibt eine Vorrichtung und ein Verfahren zur Bestimmung des Feuchtigkeitsgehalts eines Materials mit mehreren Schichten, wie z.B. Papier oder Baumwolle usw., insbesondere in Ballen-Form. Dabei werden die Ballen mittels eines Förderbands durch die Vorrichtung zur Bestimmung des Feuchtigkeitsgehalts transportiert. Die Bestimmung des Feuchtigkeitsgehaltes eines Materials erfolgt hierbei ähnlich wie oben in JP 61034448 A beschrieben.

Die DE 25 52 954 OS beschreibt eine Vorrichtung zur Feuchtemessung von räumlich ausgedehnten Proben, wie Papier, Holz, Erdboden usw., unter Verwendung eines frequenzmodulierten Mikrowellengenerators, der in Form einer Anlege- oder Eintauchsonde gestaltet ist. Auch hier beruht die Messung des Feuchtegehalts auf der Mikrowellenabsorption von in einer Probe enthaltenen Wassers, welches zu einer messbaren Dämpfung des Mikrowellensignals führt.

Die bekannten Vorrichtungen benötigen relativ aufwendige elektrische Schaltungen zur Signalverarbeitung, umfassend zumindest eine Sendeantenne und eine Empfangsantenne, weiterhin gegebenenfalls einen Phasenverschiebungs-Bestimmer, einen Dämpfungsmesser, der die Dämpfung des Mikrowellensignals misst, einen Richtkoppler usw. Die bekannten Vorrichtungen sind daher relativ empfindlich und im Hinblick auf einen Einsatz für eine Messung des Feuchtegehalts von Altpapier meist auch zu aufwendig und teuer.

Es ist daher Aufgabe der Erfindung, ein vereinfachtes Verfahren und eine vereinfachte Vorrichtung zur Bestimmung des Feuchtegehalts von Altpapier bzw. einer Altpapiercharge bereitzustellen.

Die Aufgabe wird durch ein Verfahren zur Bestimmung des Feuchtegehalts einer Altpapiercharge umfassend folgende Schritte gelöst:
- Erfassung einer Masse der Altpapiercharge;
- Beaufschlagung mindestens eines Teils der Altpapiercharge mit Mikrowellenstrahlung, welche durch mindestens einen Mikrowellengenerator erzeugt wird;
- Messung der Stromaufnahme des mindestens einen Mikrowellengenerators;
- Bestimmung des Feuchtegehalts der Altpapiercharge unter Zuhilfenahme mindestens eines vorab bestimmten Korrelationsschemas, welches eine Korrelation zwischen der gemessenen Stromaufnahme und dem Feuchtegehalt einer Altpapiercharge bereitstellt, wobei die erfasste Masse der Altpapiercharge berücksichtigt wird.

Die Aufgabe wird durch eine Vorrichtung zur Bestimmung des Feuchtegehalts einer Altpapiercharge, insbesondere zur Durchführung eines erfindungsgemäßen Verfahrens, gelöst, welche folgendes umfasst:
- eine Einrichtung zur Erfassung einer Masse der Altpapiercharge;
- eine Kammer zur mindestens teilweisen Aufnahme der Altpapiercharge;
- mindestens einen Mikrowellengenerator zur mindestens teilweisen Bestrahlung der in der Kammer zumindest teilweise aufgenommenen Altpapiercharge mit Mikrowellenstrahlung;
- mindestens ein Messgerät zur Erfassung einer Stromaufnahme des mindestens einen Mikrowellengenerators; und
- mindestens eine Steuer- und Recheneinheit zur Bestimmung des Feuchtegehalts der Altpapiercharge unter Zuhilfenahme mindestens eines vorab bestimmten und in der mindestens einen Steuer- und Recheneinheit hinterlegten Korrelationsschemas, welches eine Korrelation zwischen einer gemessenen Stromaufnahme und einem Feuchtegehalt einer Altpapiercharge bereitstellt, unter Berücksichtigung der erfassten Masse der Altpapiercharge.

Das erfindungsgemäße Verfahren und die erfindungsgemäße Vorrichtung ermöglichen eine unkomplizierte Erfassung des Feuchtegehalts von Altpapier und in Folge eine auf die Trockenmasse bezogene und vom Feuchtegehalt unabhängige Vergütung für eine angelieferte Altpapiercharge.

Eine Durchführung des erfindungsgemäßen Verfahrens und eine Bereitstellung einer erfindungsgemäßen Vorrichtung ist besonders einfach und kostengünstig möglich, da lediglich die Stromaufnahme des mindestens einen Mikrowellengenerators mit Hilfe mindestens eines Messgerätes gemessen werden muss. Es hat sich nämlich überraschend gezeigt, dass die Stromaufnahme eines Mikrowellengenerators unmittelbar von der Menge an Wasser im Altpapier bzw. von dessen Feuchtegehalt abhängig ist. Aufwendige elektrische Verschaltungen oder kostenintensive elektrische Bauteile sind bei der Messung der Stromaufnahme nicht erforderlich. Es ergibt sich eine schnelle und unkomplizierte Möglichkeit, den Feuchtegehalt einer Altpapiercharge zu bestimmen, wobei ein automatisierter und/oder kontinuierlicher Messbetrieb möglich ist.

Eine erfindungsgemäße Vorrichtung kann in kürzester Zeit erstellt werden oder in bereits bestehende Anlagen zur Anlieferung von Altpapier, die beispielsweise bereits eine Einrichtung zur Erfassung einer Masse einer angelieferten Altpapiercharge umfassen, integriert werden.

Unter einem Korrelationsschema wird insbesondere eine Kennkurve verstanden, die eine Korrelation zwischen einer gemessenen Stromaufnahme und einem Feuchtegehalt einer Altpapiercharge bereitstellt, unter Berücksichtigung der erfassten Masse der Altpapiercharge. Ein Korrelationsschema kann aber auch dadurch erstellt werden, dass eine Ermittlung des Feuchtegehalts oder der Trockenmasse von Altpapierchargen unterschiedlicher Masse erfolgt und jeweils ein Quotient aus entweder dem Feuchtegehalt und einem zugehörigen Stromaufnahmewert oder der Trockenmasse und einem zugehörigen Stromaufnahmewert in der Steuer- und Recheneinheit hinterlegt wird.

Bevorzugte Ausgestaltungen der Erfindung sind nachfolgend angegeben.

Der Feuchtegehalt des Altpapiers kann generell an der kompletten Altpapiercharge oder nur an einem, für die komplette Altpapiercharge repräsentativen Teil der Altpapiercharge bestimmt werden.

Der mindestens eine Mikrowellengenerator ist entweder relativ zur Kammer ortsfest installiert oder relativ zur Kammer beweglich installiert. Durch eine Änderung der Position des mindestens einen Mikrowellengenerators gegenüber in der Kammer angeordnetem Altpapier wird allerdings eine besonders gute Erfassung des mittleren Feuchtegehalts des in der Kammer angeordeten Altpapiers erreicht. Eine Änderung der Position des mindestens einen Mikrowellengenerators gegenüber in der Kammer angeordnetem Altpapier ist somit insbesondere dann von Vorteil, wenn der Feuchtegehalt des zu messenden Altpapiers in der Kammer stark unterschiedlich ist.

Bei dem Messgerät zur Messung der Stromaufnahme des Mikrowellengenerators handelt es sich vorzugsweise um ein Amperemeter.

Die Kammer der Vorrichtung weist vorzugsweise mindestens eine Öffnung zur Aufnahme und/oder Abgabe mindestens eines Teils der Altpapiercharge auf, welche durch mindestens eine Verschließeinheit zu öffnen oder zu schließen ist. Die mindestens eine Verschließeinheit hat die Aufgabe, die von dem mindestens einen Mikrowellengenerator während der Messung des Feuchtegehalts erzeugte Mikrowellenstrahlung in der Kammer einzuschließen, damit im Außenbereich der Kammer agierendes Bedienpersonal nicht mit der Strahlung beaufschlagt wird.

Es hat sich bewährt, wenn die Kammer eine oder zwei Öffnungen aufweist. Insbesondere hat es sich bewährt, wenn die Kammer an ihrer Oberseite eine Öffnung aufweist, in welche eine Altpapiercharge ganz oder teilweise eingefüllt oder gekippt werden kann. Dabei wird die Verschließeinheit zum Öffnen und Schließen der Öffnung beispielweise in Form mindestens einer Klappe und/oder mindestens eines Schiebers und/oder mindestens eines Rolltors realisiert.

Aber auch eine Kammer, welche seitlich eine Öffnung aufweist, durch welche eine Altpapiercharge ganz oder teilweise eingebracht und/oder wieder entfernt werden kann, hat sich bewährt. Dabei wird eine Verschließeinheit zum Öffnen und Schließen der Öffnung bevorzugt in Form mindestens einer Klappe und/oder mindestens eines Schiebers und/ oder mindestens einer Tür und/oder mindestens eines Rolltors realisiert.

Eine Kammer, welche an zwei Seiten jeweils eine Öffnung aufweist, wobei durch eine der beiden Öffnungen eine Altpapiercharge ganz oder teilweise eingebracht wird und wobei durch die andere der beiden Öffnungen das Altpapier wieder entfernt wird, hat sich ebenfalls bewährt. Dabei wird an jeder Öffnung jeweils eine Verschließeinheit, beispielweise in Form mindestens einer Klappe und/oder mindestens eines Schiebers und/ oder mindestens einer Tür und/oder mindestens eines Rolltors realisiert.

Die erfindungsgemäße Vorrichtung weist bevorzugt weiterhin mindestens eine Transporteinrichtung auf, welche mindestens einen Teil der Altpapiercharge in die und/oder aus der Kammer fördert. Dabei wird das Altpapier durch eine in der Kammer vorhandene Öffnung in die Kammer eingebracht. Bei einer derartigen Transporteinrichtung kann es sich um eine kontinuierlich arbeitende Transporteinrichtung oder um eine diskontinuierlich arbeitende Transporteinrichtung handeln. Unter einer diskontinuierlich arbeitenden Transporteinrichtung werden hierbei unter anderem Kräne, Schaufelbagger, LKWs und dergleichen verstanden. Unter einer kontinuierlich arbeitenden Transporteinrichtung werden insbesondere Förderbänder, Schwingförderer oder dergleichen verstanden.

Bevorzugt ist die mindestens eine Steuer- und Recheneinheit zur Steuerung des mindestens einen Mikrowellengenerators und/oder der mindestens einen Transporteinrichtung und/oder eines Öffnungs- oder Schließvorgangs der Kammer mittels der mindestens einen Verschließeinheit eingerichtet. Dadurch erfolgt vorzugsweise ein automatischer oder halbautomatischer Verfahrensablauf, wobei Altpapier über mindestens eine Transporteinrichtung zur Kammer transportiert, in die Kammer eingebracht, anschließend die mindestens eine Verschließeinheit betätigt und die Messung gestartet wird. Nach Durchführung der Messung des Feuchtegehalts erfolgt eine Entleerung der Kammer und der Vorgang startet erneut.

In der Kammer können sich Hilfsmittel befinden, die ein Befüllen oder Entleeren der Kammer unterstützen. So kann mindestens eine Fördereinrichtung, wie beispielsweise ein Rechen, eine Räumschaufel oder dergleichen, vorhanden sein.

Eine Erfassung der Masse des angelieferten Altpapiers kann vor, während oder nach einer Feuchtemessung an der Altpapiercharge oder eines Teils davon erfolgen.

Die Einrichtung zur Erfassung der Masse der Altpapiercharge ist dabei vorzugsweise eine elektronische Waage. Diese ist bevorzugt so angeordnet, dass die Kammer auf der elektronischen Waage angeordnet ist. Alternativ ist es möglich, die elektronische Waage in der Kammer anzuordnen. Dazu eignet sich insbesondere eine Bandwaage.

In beiden Fällen kann nach einem Befüllen der Kammer mit Altpapier unmittelbar eine Verwiegung der eingesetzten Altpapiermenge, insbesondere der ganzen Altpapiercharge, erfolgen.

Selbstverständlich ist es aber genauso möglich, eine mechanische Waage einzusetzen, um die Masse des in die Kammer eingefüllten Altpapiers zu ermitteln. Hierbei ist es allerdings notwendig, dass der gemessene Wert, z.B. vom Bedienpersonal der Vorrichtung, notiert wird, so dass eine Berücksichtigung der Masse bei der Berechnung des Feuchtegehalts erfolgen kann.

Bei dem erfindungsgemäßen Verfahren wird der Feuchtegehalt vorzugsweise mittels mindestens einer Steuer- und Recheneinheit ermittelt, in welcher die mindestens eine Kennkurve und ein Wert für die erfasste Masse der Altpapiercharge hinterlegt werden. Zur Erstellung einer geeigneten Kennkurve sind umfangreiche Vorarbeiten erforderlich, welche eine Messung der Stromaufnahme und eine sich anschließende Auswertung des Feuchtegehalts am gemessenen Altpapier mittels Wiegens im feuchten Zustand und nach einem Trocknen auf einen definierten Restfeuchtegehalt erfordert. Dabei sind Messungen an unterschiedlich feuchten Altpapierchargen vorzunehmen, um eine aussagekräftige Kennkurve zu erstellen, welche in der Lage ist, einer bestimmten Masse an Altpapier mit einem bestimmten Feuchtegehalt eine definierte Stromaufnahme zuzuordnen.

Vorzugsweise wird in dem Verfahren unter Berücksichtigung des ermittelten Feuchtegehalts und der erfassten Masse der Altpapiercharge weiterhin eine Ermittlung einer Trockenmasse der Altpapiercharge vorgenommen. In Folge wird der Trockenmasse der Altpapiercharge in der mindestens einen Steuer- und Recheneinheit bevorzugt ein Vergütungspreis für die Altpapiercharge zugeordnet.

Die mindestens eine Steuer- und Recheneinheit umfasst bevorzugt mindestens eine Ausgabeeinheit zur Ausgabe des Feuchtegehalts und/oder einer Trockenmasse und/oder eines Vergütungspreises für die Altpapiercharge oder ist mit einer solchen Ausgabeeinheit verbunden. Weiterhin können Werte wie die Masse der Altpapiercharge, die Stromaufnahme, der Zustand der Kammer (Offen - Geschlossen), die Geschwindigkeit der Transporteinrichtung(en) (Fördergeschwindigkeit für Altpapierzu- und/oder -abfuhr in/aus Kammer), der aktuelle Zustand der Verschließeinheit(en), der Füllgrad der Kammer mit Altpapier usw. ausgegeben werden.

Es hat sich bewährt, wenn der Feuchtegehalt und/oder die Trockenmasse und/oder der Vergütungspreis schließlich mittels der mindestens einen Ausgabeeinheit, welche insbesondere Bestandteil der Steuer- und Recheneinheit oder mit dieser verbunden ist, ausgegeben wird. Das Bedienpersonal kann den/die ausgegebenen Werte auslesen und einem Anlieferer von Altpapier die ihm zustehende Vergütung ausbezahlen, die unabhängig vom aktuellen Feuchtegehalt des Altpapiers ist.

Es hat sich bewährt, wenn eine erfindungsgemäße Vorrichtung weiterhin mindestens eine Schnittstelle umfasst, über welche die Steuer- und Recheneinheit mit Daten hinsichtlich einer Materialflussverwaltung und/oder eines aktuellen Altpapierpreises versorgbar ist. Insbesondere ist/sind über die mindestens eine Schnittstelle ein Internet-Anschluss und/oder ein Anschluss an eine Materialflussverwaltung in Form eines ERP-Systems (ERP: Enterprise Source Planning) realisierbar.

Gelegentlich kommt es vor, dass ein Anlieferer von Altpapier versucht, die Masse einer Altpapiercharge durch unsichtbare Zugabe von Metallschrott manipulativ zu erhöhen. Mittels der erfindungsgemäßen Vorrichtung ist es möglich, den einer Altpapiercharge zugemischten Metallschrott zu detektieren.

Dies erfolgt vorzugsweise mittels Auswertung der Stromaufnahme-Kennlinie, nachdem eine Zugabe vom Metallschrott zu einer Altpapiercharge sich in einer besonders hohen Stromaufnahme äußert. Die Überschreitung eines festgelegten Grenzwerts für die Stromaufnahme kann somit genutzt werden, um eine Zurückweisung der manipulierten Altpapiercharge zu veranlassen.

Die Figuren 1 bis 3 sollen erfindungsgemäße Vorrichtungen und erfindungsgemäße Verfahren beispielhaft erläutern. So zeigt:
- FIG 1: eine erste Vorrichtung mit einer, auf einer elektronischen Waage angeordneten Kammer im Querschnitt;
- FIG 2: eine zweite Vorrichtung mit einer, auf einer elektronischen Waage angeordneten Kammer im Querschnitt; und
- FIG 3: eine dritte Vorrichtung mit einer, in einer Kammer angeordneten elektronischen Waage im Querschnitt.

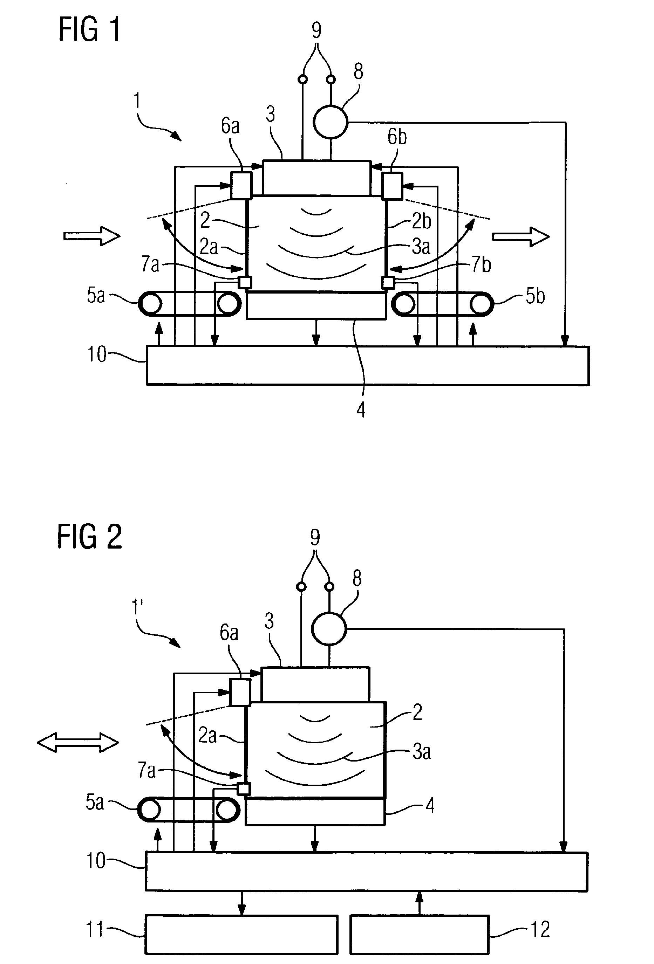

FIG 1 zeigt im Querschnitt eine erste Vorrichtung 1 zur Bestimmung des Feuchtegehalts von Altpapier mit einer, auf einer elektronischen Waage 4 angeordneten Kammer 2. Die Kammer 2 weist an ihren beiden Seiten jeweils eine Öffnung auf, welche mittels jeweils einer Verschließeinheit 2a, 2b in Form einer Klappe geöffnet und verschlossen werden können. Eine jede Klappe wird über einen Antrieb 6a, 6b bewegt, wobei der Bewegungsablauf beim Öffnung oder Schließen einer jeden Klappe über Pfeile und die maximale Auslenkung einer jeden Klappe über gestrichelte Linien dargestellt ist. Um feststellen zu können, ob eine Klappe geschlossen ist, weist die Kammer 2 weiterhin Sicherheitseinrichtungen 7a, 7b auf, welche als Sicherheitsschalter ausgeführt sind. Die Sicherheitsschalter werden jeweils mit dem Schließen einer Klappe geschlossen, wobei der Mikrowellengenerator 3 in der Kammer 2 erst dann aktiviert werden kann, sobald beide Klappen geschlossen sind und die vom Mikrowellengenerator 3 erzeugte Mikrowellenstrahlung 3a nicht mehr aus der Kammer 2 gelangen kann.

Der Mikrowellengenerator 3 ist an eine Stromversorgung 9 angeschlossen. Der Einfachheit halber ist hier lediglich ein einzelner Mikrowellengenerator 3 dargestellt. Es können aber auch mehrere bzw. auch ein oder mehrere bewegliche Mikrowellengeneratoren 3 in der Kammer 2 vorhanden sein. In der Stromzuführungsleitung zum mindestens einen Mikrowellengenerator 3 ist ein Messgerät 8 angeschlossen, welches zur Messung der Stromaufnahme des mindestens einen Mikrowellengenerators 3 geeignet ist. Bei dem Messgerät 8 handelt es sich hier um ein Amperemeter.

Im Bereich der Öffnungen in der Kammer 2 befinden sich Transporteinrichtungen 5a, 5b, hier in Form von Förderbändern. Der Pfeil links in FIG 1 stellt die Transportrichtung des Altpapiers in die Kammer 2 dar, in die es mittels der Transportvorrichtung 5a nach Betätigen der Verschließeinheit 2a bzw. nach Öffnen der Klappe transportiert wird.

Mittels der elektronischen Waage 4 wird nun die in die Kammer 2 verbrachte Masse an Altpapier, welche hier der Übersichtlichkeit halber nicht dargestellt wurde, verwogen und die Verschließeinheiten 2a, 2b derart betätigt, dass beide Öffnungen der Kammer 2 durch die Klappen verschlossen werden.

Nach Durchführung der Messung der Stromaufnahme mittels des Messgeräts 8 wird die Verschließeinheit 2b betätigt bzw. die Klappe geöffnet und das Altpapier mittels der Transporteinrichtung 5b aus der Kammer 2 befördert. Die Entleerung der Kammer 2 wird durch einen in der Kammer 2 angeordneten, nicht gesondert dargestellten Rechen unterstützt.

Weiterhin ist eine Steuer- und Recheneinheit 10 vorhanden, welche mit dem Mikrowellengenerator 3, der elektronischen Waage 4, den Antrieben 6a, 6b für die Klappen, den Sicherungseinrichtungen 7a, 7b für die Klappen, den Transporteinrichtungen 5a, 5b sowie dem Messgerät 8 verbunden ist. Die Steuer- und Recheneinheit 10 steuert dabei die Transporteinrichtungen 5a, 5b derart, dass die Transporteinrichtung 5a erst nach einem Öffnen der Klappe der Verschließeinheit 2a Altpapier in Richtung der Kammer 2 transportiert. Ist genügend Altpapier in der Kammer 2 angelangt, was insbesondere über die Laufzeit der Transporteinrichtung 5a und/oder die elektronische Waage 4 kontrollierbar ist, wird die Öffnung der Kammer 2 durch die Klappe bzw. die Verschließeinheit 2a verschlossen. Sobald beide Sicherheitseinrichtungen 7a, 7b geschlossen sind, erfolgt mittels der Steuer- und Recheneinheit 10 ein Einschalten des mindestens einen Mikrowellengenerators 3. Während der Mikrowellengenerator 3 Mikrowellen 3a aussendet, wird die Stromaufnahme des Mikrowellengenerators 3 erfasst und der Wert an die Steuer- und Recheneinheit 10 übermittelt.

Eine Mindestdauer für die Aussendung der Mikrowellenstrahlung im Mikrosekunden- bis Sekundenbereich ist von Vorteil.

In der Steuer- und Recheneinheit 10 ist eine Kennkurve hinterlegt. In Abhängigkeit von der, mittels der elektronischen Waage 4 ermittelten Masse des sich in der Kammer 2 befindlichen Altpapiers wird auf Basis der Kennkurve einer bestimmten, mittels des Messgerätes 8 gemessenen Stromaufnahme ein bestimmter Wert für den Feuchtegehalt des Altpapiers zuordnet.

Zur Erstellung einer geeigneten Kennkurve sind, wie oben bereits dargelegt, umfangreiche Vorarbeiten erforderlich, welche eine Messung der Stromaufnahme und eine sich anschließende Auswertung des Feuchtegehalts am gemessenen Altpapier mittels Wiegens im feuchten Zustand und nach einem Trocknen auf einen definierten Restfeuchtegehalt erfordert. Dabei sind Messungen an unterschiedlich feuchten Altpapierchargen vorzunehmen, um eine aussagekräftige Kennkurve zu erstellen, welche einer bestimmten Masse an Altpapier mit einem bestimmten Feuchtegehalt eine definierte Stromaufnahme zuordnen kann.

Nach Durchführung der Messung wird der Mikrowellengenerator 3 abgeschaltet und die Verschließeinrichtung 2b betätigt bzw. die Klappe geöffnet. Nun wird das Altpapier mittels des hier nicht gezeigten Rechens aus der Kammer 2 in Richtung der Transporteinrichtung 5b befördert und mittels der Transporteinrichtung 5b abtransportiert.

Eine in die Steuer- und Recheneinheit 10 integrierte Ausgabeeinheit gibt insbesondere die Werte für die Masse an Altpapier, den Feuchtegehalt, und die zu zahlende Vergütung für das Bedienpersonal lesbar aus. Insbesondere können noch weitere Werte, wie die Trockenmasse des Altpapiers, die Dauer der Messung usw., ausgegeben werden.

FIG 2 zeigt eine zweite Vorrichtung 1' mit einer, auf einer elektronischen Waage 4 angeordneten Kammer 2 im Querschnitt.

Für gleiche Teile der ersten Vorrichtung 1 gemäß FIG 1 und der zweiten Vorrichtung 1' gemäß FIG 2 sind gleiche Bezugszeichen verwendet. Im Unterschied zu FIG 1 weist hier die Kammer 2 lediglich eine Öffnung auf, die mittels einer Verschließeinheit 2a in Form einer Klappe geöffnet oder verschlossen werden kann. Weiterhin ist die Transporteinrichtung 5a derart eingerichtet, dass Altpapier zur Kammer 2 hin und von der Kammer 2 weg gefördert werden kann (siehe Doppelpfeil links in der FIG 2). Im Übrigen funktioniert die zweite Vorrichtung 1' analog zur ersten Vorrichtung 1. Zusätzlich ist hier noch die Steuer- und Recheneinheit 10 mit einer Materialflussverwaltung in Form eines SAP ERP-Systems 11 und dem Internet 12 verbunden. Über das Internet 12 wird die Steuer- und Recheneinheit 10 mit aktuellen Weltmarktpreisen für Altpapier versorgt. Die ermittelte Trockenmasse des angelieferten Altpapiers wird über die Materialflussverwaltung in die Bedarfsplanung und Materialverwaltung, beispielweise einer das Altpapier ankaufenden Papierfabrik, einbezogen.

Die Verbindung zur Materialflussverwaltung in Form eines SAP ERP-Systems 11 und dem Internet 12 ist natürlich in gleicher Weise auch für die erste Vorrichtung 1 gemäß FIG 1 von Vorteil.

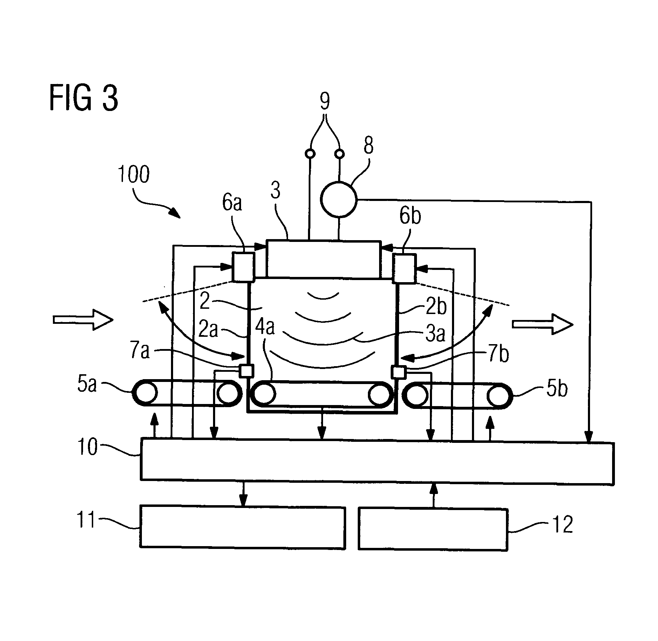

FIG 3 zeigt eine dritte Vorrichtung 100 mit einer in einer Kammer 2 angeordneten elektronischen Waage 4a im Querschnitt. Für gleiche Teile der ersten Vorrichtung 1 gemäß FIG 1 und der dritten Vorrichtung 100 gemäß FIG 3 sind gleiche Bezugszeichen verwendet. Die elektronische Waage 4a ist als Bandwaage ausgeführt, die gleichzeitig als Förderband agiert, um Altpapier aus der Kammer 2 in Richtung der Transporteinrichtung 5b zu fördern. Im Übrigen arbeitet die dritte Vorrichtung 100 analog zur ersten Vorrichtung 1. Zusätzlich ist auch hier die Steuer- und Recheneinheit 10 mit einer Materialflussverwaltung in Form eines SAP ERP-Systems 11 und dem Internet 12 verbunden. Über das Internet 12 wird die Steuer- und Recheneinheit 10 mit aktuellen Weltmarktpreisen für Altpapier versorgt. Die ermittelte Trockenmasse des angelieferten Altpapiers wird über die Materialflussverwaltung in die Bedarfsplanung und Materialverwaltung, beispielweise einer das Altpapier ankaufenden Papierfabrik, einbezogen.

Die in den FIGen 1 bis 3 dargestellten Vorrichtung und zugehörigen Verfahren sind lediglich beispielhaft aufgeführt. Ein Fachmann ist aber jederzeit in der Lage, eine erfindungsgemäße Vorrichtung und ein erfindungsgemäßes Verfahren abweichend von den dargestellten Ausführungsbeispielen zu realisieren. So kann beispielsweise eine Kammer mit Rolltor eingesetzt werden, die Einrichtung zur Bestimmung der Masse an Altpapier von der Kammer getrennt angeordnet werden, andere Transporteinrichtungen verwendet werden und dergleichen mehr.

## Patentansprüche

1. Verfahren zur Bestimmung des Feuchtegehalts einer Altpapiercharge, umfassend folgende Schritte:
- Erfassung einer Masse der Altpapiercharge;
- Beaufschlagung mindestens eines Teils der Altpapiercharge mit Mikrowellenstrahlung (3a), welche durch mindestens einen Mikrowellengenerator (3) erzeugt wird;
- Messung der Stromaufnahme des mindestens einen Mikrowellengenerators (3);
- Bestimmung des Feuchtegehalts der Altpapiercharge unter Zuhilfenahme mindestens eines vorab bestimmten Korrelationschemas, welches eine Korrelation zwischen der gemessenen Stromaufnahme und dem Feuchtegehalt einer Altpapiercharge bereitstellt, wobei die erfasste Masse der Altpapiercharge berücksichtigt wird.

2. Verfahren nach Anspruch 1,
**dadurch gekennzeichnet, dass** der Feuchtegehalt mittels mindestens einer Steuer- und Recheneinheit (10) ermittelt wird, in welcher das mindestens eine Korrelationsschema und ein Wert für die erfasste Masse der Altpapiercharge hinterlegt werden.

3. Verfahren nach Anspruch 1 oder Anspruch 2,
**dadurch gekennzeichnet, dass** unter Berücksichtigung des ermittelten Feuchtegehalts und der erfassten Masse der Altpapiercharge weiterhin eine Ermittlung einer Trockenmasse der Altpapiercharge erfolgt.

4. Verfahren nach den Ansprüchen 2 und 3,
**dadurch gekennzeichnet, dass** der Trockenmasse der Altpapiercharge in der mindestens einen Steuer- und Recheneinheit (10) ein Vergütungspreis für die Altpapiercharge zugeordnet wird.

5. Verfahren nach einem der Ansprüche 2 bis 4,
**dadurch gekennzeichnet, dass** der Feuchtegehalt und/oder die Trockenmasse und/oder der Vergütungspreis mittels mindestens einer Ausgabeeinheit ausgegeben wird.

6. Vorrichtung (1, 1', 100) zur Bestimmung des Feuchtegehalts einer Altpapiercharge, insbesondere zur Durchführung eines Verfahrens nach einem der Ansprüche 1 bis 5, umfassend
- eine Einrichtung zur Erfassung einer Masse der Altpapiercharge;
- eine Kammer (2) zur mindestens teilweisen Aufnahme der Altpapiercharge;
- mindestens einen Mikrowellengenerator (3) zur mindestens teilweisen Bestrahlung der in der Kammer zumindest teilweise aufgenommenen Altpapiercharge mit Mikrowellenstrahlung (3a);
- mindestens ein Messgerät (8) zur Erfassung einer Stromaufnahme des mindestens einen Mikrowellengenerators (3); und
- mindestens eine Steuer- und Recheneinheit (10) zur Bestimmung des Feuchtegehalts der Altpapiercharge unter Zuhilfenahme mindestens eines vorab bestimmten und in der mindestens einen Steuer- und Recheneinheit (10) hinterlegten Korrelationsschemas, welches eine Korrelation zwischen einer gemessenen Stromaufnahme und einem Feuchtegehalt einer Altpapiercharge bereitstellt, unter Berücksichtigung der erfassten Masse der Altpapiercharge.

7. Vorrichtung nach Anspruch 6,
**dadurch gekennzeichnet, dass** die Vorrichtung (1, 1', 100) weiterhin mindestens eine Transporteinrichtung (5a, 5b) umfasst, welche mindestens einen Teil der Altpapiercharge in die und/oder aus der Kammer (2) fördert.

8. Vorrichtung nach Anspruch 6 oder Anspruch 7,
**dadurch gekennzeichnet, dass** die Kammer (2) mindestens eine Öffnung zur Aufnahme und/oder Angabe mindestens eines Teils der Altpapiercharge aufweist, welche durch mindestens eine Verschließeinheit (2a, 2b) zu öffnen oder zu schließen ist.

9. Vorrichtung nach einem der Ansprüche 6 bis 8,
**dadurch gekennzeichnet, dass** die mindestens eine Steuer- und Recheneinheit (10) zur Steuerung des mindestens einen Mikrowellengenerators (3) und/oder der mindestens einen Transporteinrichtung (5a, 5b) und/oder eines Öffnungs- oder Schließvorgangs der Kammer (2) mittels der mindestens einen Verschließeinheit (2a, 2b) eingerichtet ist.

10. Vorrichtung nach einem der Ansprüche 6 bis 9,
**dadurch gekennzeichnet, dass** die mindestens eine Steuer- und Recheneinheit (10) mindestens eine Ausgabeeinheit zur Ausgabe des Feuchtegehalts und/oder einer Trockenmasse und/oder eines Vergütungspreises für die Altpapiercharge umfasst oder mit einer solchen Ausgabeeinheit verbunden ist.

11. Vorrichtung nach einem der Ansprüche 6 bis 10,
**dadurch gekennzeichnet, dass** die Einrichtung zur Erfassung der Masse der Altpapiercharge eine elektronische Waage (4) ist und dass die Kammer (2) auf der elektronischen Waage angeordnet ist.

12. Vorrichtung nach einem der Ansprüche 6 bis 10,
**dadurch gekennzeichnet, dass** die Einrichtung zur Erfassung der Masse der Altpapiercharge eine elektronische Waage (4a) ist und/oder dass die Waage in der Kammer (2) angeordnet ist.

13. Vorrichtung nach einem der Ansprüche 6 bis 12,
**dadurch gekennzeichnet, dass** die Vorrichtung (1, 1', 100) mindestens eine Schnittstelle umfasst, über welche die Steuer- und Recheneinheit (10) mit Daten hinsichtlich einer Materialflussverwaltung und/oder eines aktuellen Altpapierpreises versorgbar ist.

## Claims

1. Method for determining the moisture content of a batch of waste paper, comprising the following steps:
- Measuring the mass of the batch of waste paper;
- Applying microwave radiation (3a) generated by at least one microwave generator (3) to at least some of the batch of waste paper;
- Measuring the power consumption of the at least one microwave generator (3);
- Determining the moisture content of the batch of waste paper with the aid of at least one previously determined correlation system, which provides a correlation between the measured power consumption and the moisture content of a batch of waste paper, with the measured mass of the batch of waste paper being taken into account.

2. Method according to claim 1,
**characterised in that** the moisture content is determined by means of at least one control and computation unit (10), in which the at least one correlation system and a value for the measured mass of the batch of waste paper are stored.

3. Method according to claim 1 or claim 2,
**characterised in that** a dry mass of the batch of waste paper is also determined taking into account the determined moisture content and the measured mass of the batch of waste paper.

4. Method according to claims 2 and 3,
**characterised in that** a payment price for the batch of waste paper is assigned to the dry mass of the batch of waste paper in the at least one control and computation unit (10).

5. Method according to one of claims 2 to 4,
**characterised in that** the moisture content and/or the dry mass and/or the payment price is/are output by means of at least one output unit.

6. Apparatus (1, 1', 100) for determining the moisture content of a batch of waste paper, in particular for implementing a method according to one of claims 1 to 5, comprising
- A facility for measuring a mass of the batch of waste paper;
- A chamber (2) to accommodate at least some of the batch of waste paper;
- At least one microwave generator (3) for irradiating with microwave radiation (3a) at least some of the batch of waste paper at least some of which is accommodated in the chamber;
- At least one measuring device (8) for measuring a power consumption of the at least one microwave generator (3); and
- At least one control and computation unit (10) for determining the moisture content of the batch of waste paper with the aid of at least one previously determined correlation system stored in the control and computation unit (10), which provides a correlation between a measured power consumption and a moisture content of a batch of waste paper, taking into account the measured mass of the batch of waste paper.

7. Apparatus according to claim 6,
**characterised in that** the apparatus (1, 1', 100) also comprises at least one transport facility (5a, 5b), which conveys at least some of the batch of waste paper into and/or out of the chamber (2).

8. Apparatus according to claim 6 or claim 7,
**characterised in that** the chamber (2) has at least one opening for receiving and/or outputting at least some of the batch of waste paper, said opening being able to be opened or closed by at least one closing unit (2a, 2b).

9. Apparatus according to one of claims 6 to 8,
**characterised in that** the at least one control and computation unit (10) is set up to control the at least one microwave generator (3) and/or the at least one transport facility (5a, 5b) and/or an opening or closing operation of the chamber (2) by means of the at least one closing unit (2a, 2b).

10. Apparatus according to one of claims 6 to 9,
**characterised in that** the at least one control and computation unit (10) comprises at least one output unit for outputting the moisture content and/or a dry mass and/or a payment price for the batch of waste paper or is connected to such an output unit.

11. Apparatus according to one of claims 6 to 10,
**characterised in that** the facility for measuring the mass of the batch of waste paper is an electronic scale (4) and the chamber (2) is disposed on the electronic scale (4).

12. Apparatus according to one of claims 6 to 10,
**characterised in that** the facility for measuring the mass of the batch of waste paper is an electronic scale (4a) and/or the scale is disposed in the chamber (2).

13. Apparatus according to one of claims 6 to 12,
**characterised in that** the apparatus (1, 1', 100) comprises at least one interface, by way of which the control and computation unit (10) can be supplied with data relating to material flow management and/or a current waste paper price.

## Revendications

1. Procédé de détermination de la teneur en humidité d'une charge de vieux papier, comprenant les stades suivants :
- on détermine une masse de la charge de vieux papier ;
- on soumet au moins une partie de la charge de vieux papier à du rayonnement (3a) micro-onde, qui est produit par au moins un générateur ( 3 ) de micro-onde ;
- on mesure l'absorption de courant du au moins un générateur ( 3 ) de micro-onde ;
- on détermine la teneur en humidité de la charge de vieux papier en s'aidant d'au moins un schéma de corrélation déterminé auparavant, qui donne une corrélation entre l'absorption de courant mesuré et la teneur en humidité d'une charge de vieux papier, la masse déterminée de la charge de vieux papier étant prise en compte.

2. Procédé suivant la revendication 1,
**caractérisé en ce qu'**on détermine la teneur en humidité au moyen d'une unité (10) de commande et informatique, dans laquelle le au moins un schéma de corrélation et une valeur de la masse déterminée de la charge de vieux papier sont mémorisés.

3. Procédé suivant la revendication 1 ou revendication 2,
**caractérisé en ce qu'**en tenant compte de la teneur en humidité déterminée et de la masse déterminée de la charge de vieux papier, on effectue, en outre, une détermination d'une masse sèche de la charge de vieux papier.

4. Procédé suivant les revendications 2 et 3,
**caractérisé en ce qu'**il est affecté à la masse sèche de la charge de vieux papier, dans la au moins une unité ( 10 ) de commande et informatique, un prix de valorisation de la charge de vieux papier.

5. Procédé suivant l'une des revendications 2 à 4, **caractérisé en ce qu'**on sort la teneur en humidité et/ou la masse sèche et/ou le prix de valorisation au moyen d'au moins une unité de sortie.

6. Dispositif ( 1, 1', 100 ) de détermination de la teneur en humidité d'une charge de vieux papier, notamment pour effectuer un procédé suivant l'une des revendications 1 à 5, comprenant :
- un dispositif de détermination d'une masse de la charge de vieux papier ;
- une chambre ( 2 ) de réception, au moins en partie, de la charge de vieux papier ;
- au moins un générateur ( 3 ) de micro-onde pour exposer, au moins en partie, la charge de vieux papier reçue, au moins en partie, dans la chambre à du rayonnement ( 3a ) micro-onde ;
- au moins un appareil ( 8 ) de mesure pour la détermination d'une absorption de courant du au moins un générateur ( 3 ) de micro-onde ; et
- au moins une unité ( 10 ) de commande et informatique pour la détermination de la teneur en unité de la charge de vieux papier en s'aidant d'au moins un schéma de corrélation déterminé auparavant et mémorisé dans la au moins une unité ( 10 ) de commande et informatique, schéma qui donne une corrélation entre une absorption de courant mesurée et une teneur en humidité d'une charge de vieux papier, en tenant compte de la masse déterminée de la charge de vieux papier.

7. Dispositif suivant la revendication 6,
**caractérisé en ce que** le dispositif ( 1, 1', 100 ) comprend, en outre, au moins un dispositif ( 5a, 5b ) de transport, qui transporte au moins une partie de la charge de vieux papier dans la chambre ( 2 ) et/ou hors de la chambre ( 2 ).

8. Dispositif suivant la revendication 6 ou revendication 7,
**caractérisé en ce que** la chambre ( 2 ) comporte au moins une ouverture pour la réception et/ou la sortie d'au moins une partie de la charge de vieux papier, ouverture qui peut être ouverte ou peut être fermée par au moins une unité ( 2a, 2b ) de fermeture.

9. Dispositif suivant l'une des revendications 6 à 8,
**caractérisé en ce que** la au moins une unité ( 10 ) de commande et informatique est conçue pour la commande du au moins un générateur ( 3 ) de micro-onde et/ou du au moins un dispositif ( 5a, 5b ) de transport et/ou d'une opération d'ouverture ou de fermeture de la chambre ( 2 ) au moyen de la au moins une unité ( 2a, 2b ) de fermeture.

10. Dispositif suivant l'une des revendications 6 à 9,
**caractérisé en ce que** la au moins une unité ( 10 ) de commande et informatique comprend au moins une unité de sortie, pour sortir la teneur en humidité et/ou une masse sèche et/ou une prix de valorisation de la charge de vieux papier, ou est reliée à une unité de sortie de ce genre.

11. Dispositif suivant l'une des revendications 6 à 10,
**caractérisé en ce que** le dispositif de détermination de la masse de la charge de vieux papier est une bascule ( 4 ) électronique et **en ce que** la chambre ( 2 ) est disposée sur la bascule électronique.

12. Dispositif suivant l'une des revendications 6 à 10, **caractérisé en ce que** le dispositif de détermination de la masse de la charge de vieux papier est une bascule (4a) électronique et/ou **en ce que** la bascule est disposée dans la chambre ( 2 ).

13. Dispositif suivant l'une des revendications 6 à 12, **caractérisé en ce que** le dispositif ( 1, 1' , 100) comprend au moins une interface, par laquelle l'unité ( 10 ) de commande et informatique peut être alimentée en données concernant une gestion de flux de matière et/ou un prix actuel du vieux papier.
